# EUROPEAN PATENT APPLICATION

(11) **EP 4 269 982 A1**
(43) Date of publication of application: **01.11.2023**
(21) Application number: 21910595.4
(22) Date of filing: 16.12.2021
(51) Int. Cl.: G01N 1/04, G01N 33/48

(54) **FECES-SAMPLING CONTAINER**

(30) Priority: 23.12.2020 JP 2020213817
(71) Applicant: Minaris Medical Co., Ltd., Tokyo 104-6004 (JP)
(72) Inventor: SHIMADA, Yasumasa, Tokyo 104-6004 (JP)
(74) Representative: Vossius & Partner Patentanwälte Rechtsanwälte mbB
(86) International application number: PCT/JP2021/046534
(87) International publication number: WO 2022/138447

(57) **Abstract**

Provided is a feces sampling container that reduces variation in feces sampling amount with an excellent quantification. In addition, provided is a method for collecting feces that reduces variation in feces sampling amount with an excellent quantification. Provided is a feces sampling container including: a feces sampling stick 10 that includes a stick part 12 and a feces sampling part 17 with axially-inclined grooves 17a near an end of the stick part 12; and a container body that houses the feces sampling stick 10 and includes a leveling part to scrape off feces at a position through which the feces sampling part 17 passes when the feces sampling stick 10 is housed in the container body.

## Description

### Technical Field

The present invention relates to a feces sampling container, more particularly to a feces sampling container that reduces variation in feces sampling amount with an excellent quantification.

### Background Art

A so-called fecal occult blood test that tests hemoglobin in feces excreted from animals including humans is very useful for diagnosing various diseases such as tumors of a lower gastrointestinal tract such as the large intestine; thus, this test is often used in a clinical lavatory test. A clinical laboratory test requires quantitative feces collection and suspension of the feces in an appropriate buffer, and various feces sampling containers to accomplish this testing by simple, hygienic and precise means and to allow hygienic storage and transportation after the feces collection have been developed and various reports regarding such containers have been published.

For example, Patent Document 1 discloses a feces sampling container including a feces sampling stick, a container body, and a fitting body fitted inside the container body, wherein the feces sampling stick has a gripping part on one side and a stick part on the other side, and a feces sampling part is provided near a front end of the stick part; the container body has an opening into which the fitting body is fitted on one side and a bottom on the other side; a fecal solvent storing part for storing a fecal solvent is formed in a space between a lower part of the container body and below the fitting body; the fitting body includes a cylindrical guiding part into which the stick part of the feces sampling stick can be inserted, a first leveling hole used for removing excess feces that is provided to the cylindrical guiding part, and a second leveling hole used for further removing excess feces that is provided below the first leveling hole; and a collected feces detection domain is formed to the cylindrical guiding part adjacent to an upper end of the first leveling hole. However, Patent Document 1 discloses no axially-inclined grooves of the feces sampling part, described later.

Patent Document 2 discloses a feces sampling tube including: a substantially cylindrical container that is opened at one end and is closed at the other end to accommodate a culture medium; a cap detachably attached to an open end of the container; and a feces sampling body integrally fixed to the cap, wherein a plurality of recessed grooves are formed on the surface of the feces sampling body, the plurality of recessed grooves formed on the surface of the feces sampling body are formed in a spiral shape in a direction inclined at a predetermined angle relative to the axial direction of the feces sampling body. However, Patent Document 2 discloses no leveling part to scrape off the feces, described later.

In addition, Patent Document 3 discloses a feces sampling container consisting of: a container body; and a feces sampling stick that includes a gripping part on one side and a stick part on the other side, and the stick part has a feces sampling part on near a front end of thereof, wherein the container body includes: an opening part into which the feces sampling part of the feces sampling stick is inserted; and a feces storing chamber where a desiccant is enclosed inside, the feces sampling part inserted from the opening and holding a feces sample comes into contact with the desiccant to dry the feces sample held by the feces sampling part and store this feces sample in a dried state in the desiccant.

### Prior Art Documents

### Patent Documents

**Patent Document 1:** International Publication No. WO2010/067534
**Patent Document 2:** Japanese unexamined Patent Application Publication No. 2009-276311
**Patent Document 3:** International Publication No. WO2017/104132

### Summary of the Invention

### Object to be Solved by the Invention

In case of a test using an antigen-antibody reaction against an antigen contained in a feces sample, it is required to secure a constant amount of a feces sample regardless of the properties of the feces to be tested, and it is also required to keep constant the feces concentration in a feces sample buffer; however, it is difficult to conduct a quantitative test using the above feces sampling containers.

To cope with this, the present disclosure provides a feces sampling container that reduces variation in feces sampling amount with an excellent quantification. In addition, the present disclosure provides a method for collecting feces that reduces variation in feces sampling amount with an excellent quantification.

### Means to Solve the Object

The present invention includes various embodiments. Examples of the embodiments are provided below. The present invention is not limited to the following embodiments. The following embodiments can be implemented alone or in combination.

Embodiment 1 at least includes a feces sampling stick that includes a stick part and a feces sampling part having an axially-inclined groove near an end of the stick part; and a container body that houses the feces sampling stick and includes a leveling part to scrape off feces at a position through which the feces sampling part passes when housing the feces sampling stick in the container body.

In Embodiment 1, excess feces adhering to the feces sampling stick is scraped off and removed by the leveling part. In addition, even when filling of the feces into the axially-inclined grooves is insufficient and a gap is generated in the axially-inclined grooves during feces sampling, the feces is pushed into the axially-inclined grooves when the excess feces is scraped off and removed by the leveling part, to thereby fill all the feces sampling grooves with the feces. As a result, it is possible to reduce variation in feces sampling amount.

In Embodiment 1, when the feces sampling stick is housed in the feces sampling container, the feces sampling part may pass through the leveling part while the feces sampling part does not rotate, or the feces sampling part may pass through the leveling part while the feces sampling part rotates. In Embodiment 1, the container body may be separatable into a plurality of members. The plurality of members may include a container outer frame and a fitting body that is fitted inside the container outer frame. When the container body is separatable into a plurality of members, the leveling part and the cylindrical guiding part described later may be collectively provided as one member among the plurality of members or may be separately provided as the plurality of members.

Embodiment 2, in the feces sampling container of Embodiment 1, at least includes that the stick part of the feces sampling stick has a screw part, and the container body includes a screw hole into which the screw part is screwed.

In general, when the feces sampling part of the feces sampling stick is pushed against the feces, in order to conduct the feces sampling even if the feces sampling part is pushed against the feces in any direction, the feces sampling grooves are required to be arranged at 360° around the feces sampling part. However, even in case of 360° arrangement of the feces sampling grooves, when the feces sampling stick is pushed against the feces only in one direction, the feces can be sampled only in the feces sampling groove in this direction of the feces sampling stick. Therefore, although the feces sampling can be conducted, variation in feces sampling amount is caused. To cope with this, it is considered that even in case of pushing the feces sampling stick against the feces only in one direction, there are required a function of filling all the feces sampling grooves arranged at 360° around the whole circumference of the feces sampling part with the feces, a structure of facilitating the filling of the feces sampling grooves with the feces, or a function of forcibly filling the feces sampling grooves with the feces.

Embodiment 2 is configured such that the feces sampling stick is screwed into the container body. In Embodiment 2, even in case of pushing the feces sampling stick against the feces in only one direction, when the feces sampling stick is housed in the container body thereafter, excess feces scraped off and removed by the leveling part is pushed into the axially-inclined grooves along with the rotation of the feces sampling part caused by the screwing, to thereby fill all the feces sampling grooves with the feces. As a result, variation in feces sampling amount is reduced.

Embodiment 3, in the feces sampling container of Embodiment 2, at least includes that the feces sampling stick includes the screw part on a base portion of the other end of the stick part. In Embodiment 3, the feces sampling stick may be provided with a gripping part so as to facilitate gripping of the feces sampling stick when a feces sampling operator conducts the feces sampling.

Embodiment 4, in the feces sampling container of Embodiments 1 to 3, at least includes that, when the feces sampling stick is screwed into the container body by 1.5 turns or more, a ratio of a distance of insertion of the feces sampling stick to a distance from one end to the other end in an axial direction of the axially-inclined groove is 1:1. In Embodiment 4, a distance in the axial direction of the axially-inclined grooves, and the number of rotations when the feces sampling part provided with the axially-inclined grooves passes through the leveling part of the container body are set appropriately. Hence, the axially-inclined grooves can be easily filled with the feces along with the rotation of the feces sampling stick. As a result, variation in feces sampling amount is reduced.

Embodiment 5, in the feces sampling container of Embodiments 1 to 4, at least includes a plurality of axially-inclined grooves.

Embodiment 6, in the feces sampling container of Embodiments 1 to 5, at least includes that a groove depth of the axially-inclined groove is gradually decreased from a front end side toward a base end side of the stick part. Here, the front end side of the stick part is one of the both end sides of the stick part, closer to the side where the stick part has the feces sampling part near the end of the stick part. The base end side of the stick part is the other of the both end sides of the stick part, opposite to the side where the stick part has the feces sampling part near the end of the stick part, and for example, the end side on which the screw part is provided in Embodiment 3. In Embodiment 6, since the groove depth of the axially-inclined groove is gradually decreased from the front end side toward the base end side of the stick part, the axially-inclined grooves can be easily filled with the feces along with the rotation of the feces sampling stick. As a result, variation in feces sampling amount is reduced.

Embodiment 7, in the feces sampling container of Embodiments 1 to 6, at least includes that the axially-inclined groove has a spiral structure at an angle of 3° or more and 4° or less along the outer circumference of the stick part. In Embodiment 7, since the above spiral structure is a spiral structure at an angle of 3° or more and 4° or less, the axially-inclined grooves can be easily filled with the feces along with the rotation of the feces sampling stick. As a result, variation in feces sampling amount is reduced.

Embodiment 8, in the feces sampling container of Embodiments 1 to 7, at least includes that the container body includes a cylindrical guiding part that guides the feces sampling part to the leveling part, and the cylindrical guiding part includes a protruding member on an inner wall of an end on a leveling part side of the cylindrical guiding part. In Embodiment 8, when being inserted into the cylindrical guiding part, the feces sampling stick is inserted from above toward the bottom while rotating. At this time, the excess feces adhering to the feces sampling part is removed by the leveling part. The removed excess feces remains at a boundary between the end on the leveling part side of the cylindrical guiding part and the leveling part, and this remaining feces is accumulated on the side surface of the protruding member along with the rotation of the feces sampling stick. Here, further rotation of the feces sampling stick causes the protruding member to eventually and forcibly push the feces into the axially-inclined grooves of the feces sampling stick. As a result, variation in feces sampling amount is reduced.

Embodiment 9, in the feces sampling container of Embodiments 1 to 8, at least includes that the stick part is a round stick part having a constant diameter, and the round stick part includes: a hemispherical part; a circumferentially recessed part; and an upper disk part sequentially from a front end side toward a base end side of the stick part. Here, the front end side of the stick part is one of the both end sides of the stick part, closer to the side where the stick part has the feces sampling part near the end of the stick part. The base end side of the stick part is the other of the both end sides of the stick part, opposite to the side where the stick part has the feces sampling part near the end of the stick part, and for example, the end side on which the screw part is provided in Embodiment 3. In Embodiment 9, the circumferentially recessed part serves as a space where the feces is held, as in the axially-inclined grooves. The circumferentially recessed part can be used for adjusting desired amount of the feces to be collected by adjusting the depth of the recessed part and the thickness in the axial direction of the feces sampling stick during forming of the feces sampling container. In addition, part of the feces adhering to the side surface of the feces sampling stick is caught in the recessed part, to thereby prevent the collected feces from dropping until the feces after the feces sampling is housed in the container body. As a result, variation in feces sampling amount is reduced.

Embodiment 10, in the feces sampling container of Embodiment 9, at least includes that the hemispherical part has a polished surface. In Embodiment 10, since the hemispherical part has a polished surface, excess feces is prevented from adhering to the front end of the feces sampling stick. As a result, variation in feces sampling amount is reduced.

Embodiment 11, in the feces sampling container of Embodiment 9 or 10, at least includes that the circumferentially recessed part includes one or more ribs. In Embodiment 11, the rib or ribs prevent the leveling part or the second leveling part described later from biting into the circumferentially recessed part. Accordingly, the rib or ribs prevent the feces held in the circumferentially recessed part from being decreased. As a result, variation in feces sampling amount is reduced.

Embodiment 12, in the feces sampling container of Embodiments 9 to 11, at least includes that the stick part includes a lower disk part between the hemispherical part and the circumferentially recessed part.

Embodiment 13, in the feces sampling container of Embodiment 12, at least includes that an axial thickness of the lower disk part is 0.5 mm or more and 1.5 mm or less.

Embodiment 14, in the feces sampling container of Embodiments 9 to 13, at least includes a gap between the protruding member and the round stick part. In Embodiment 14, due to surface tension generated by the presence of the gap, loose feces or watery feces is naturally held in the axially inclined grooves. As a result, variation in feces sampling amount is reduced.

Embodiment 15, in the feces sampling container of Embodiments 9 to 14, at least includes that the round stick part includes a reduced-diameter step having a cutout on a base end side of the round stick part. The feces adhering to the inner wall of the cylindrical guiding part is scraped off by an axial edge of the cutout, and the feces thus scraped-off drops to a boundary between the end on the leveling part of the cylindrical guiding part and the leveling part. Thereafter, the feces having dropped is used together with the excess feces scraped-off by the leveling part as feces to be pushed into the axially-inclined grooves along with the rotation of the feces sampling stick. Alternatively, the feces having dropped is used together with the excess feces scraped-off by the leveling part as feces to be forcibly pushed into the axially-inclined grooves of the feces sampling stick by the protruding member on the inner wall of the end on the leveling part side of the cylindrical guiding part. Accordingly, even in case of having less feces removed by the leveling part, it is possible to prepare sufficient amount of feces to be pushed into the axially-inclined grooves by the protruding member. As a result, variation in feces sampling amount is reduced.

Embodiment 16, in the feces sampling container of Embodiments 9 to 15, at least further includes a second leveling part formed so that a hole diameter is reduced to be not more than a diameter of the round stick part. In Embodiment 16, even if the excess feces is insufficiently removed by the leveling part of the container body, the hole diameter of the second leveling part is reduced to be not more than the diameter of the round stick part, to thereby sufficiently remove the excess feces by the second leveling part. As a result, variation in feces sampling amount is reduced.

Embodiment 17, in the feces sampling container of Embodiment 16, at least includes that the container body includes a container outer frame and a fitting body fitted inside the container outer frame; the fitting body includes a separator made of a polyethylene (PE) or elastomer material, the separator fittable to a bottom of the fitting body; and the separator includes the second leveling part. By using a PE or elastomer material, which is softer than a polypropylene (PP) material or other materials, it is possible to enhance the function of removing the excess feces while maintaining insertability of the feces sampling stick through the second leveling part having a hole diameter reduced to be not more than a diameter of the round stick part. As a result, variation in feces sampling amount is reduced.

Embodiment 18 is a method for collecting feces and at least includes collecting feces using the feces sampling container according to Embodiments 1 to 17. By using the feces sampling container of Embodiments 1 to 17, it is possible to collect feces that reduces variation in feces sampling amount with an excellent quantification.

### Effect of the Invention

According to the present disclosure, provided is a feces sampling container that reduces variation in feces sampling amount with an excellent quantification. In addition, according to the present disclosure, provided is a method for collecting feces that reduces variation in feces sampling amount with an excellent quantification.

### Brief Description of Drawings

[Figure 1] Figure 1 is a view showing a feces sampling part of a feces sampling stick.
[Figure 2] Figure 2 is a perspective view of a longitudinal section of a leveling part for feces in a feces sampling container.
[Figure 3] Figure 3 is an explanatory view of a function of filling feces by a protruding member.
[Figure 4] Figure 4 is an explanatory view of a function capable of sampling feces even with a high moisture content.
[Figure 5] Figure 5 is an explanatory view showing how feces at a front end of a feces sampling stick moves when the feces is sampled using only a part of the feces sampling stick (front end of the feces sampling part).
[Figure 6] Figure 6 is an explanatory view showing a function of scraping off the feces on an inner wall of a cylindrical guiding part by reduced-diameter steps with cutouts.
[Figure 7] Figure 7 is a view showing a detachable separator provided with a second leveling part.
[Figure 8] Figure 8 is a view showing an entire feces sampling stick on the left, and a round stick part of the feces sampling stick on the right, respectively.
[Figure 9] Figure 9 is a view showing a stick part of the feces sampling stick including ribs provided to a circumferentially recessed part.
[Figure 10] Figure 10 is a longitudinally sectional view of the feces sampling container.

### Mode of Carrying Out the Invention

Hereinafter, a feces sampling container of a "fecal solvent type" as one embodiment of the present invention will be described with reference to the drawings, the feces sampling container including: a feces sampling stick; a container body including a container outer frame and a fitting body fitted inside the container outer frame; and a fecal solvent storing part. The present invention is not limited to the following embodiments. The following embodiments can be implemented alone or in combination.

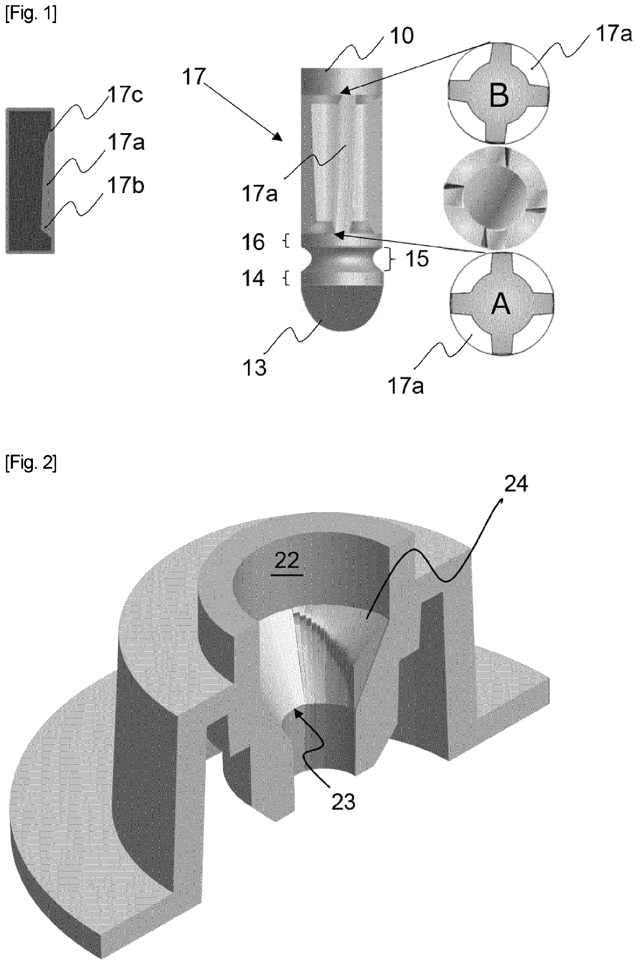

Figure 1 (middle) is a front view of a feces sampling part (17) of the feces sampling stick (10). Figure 1 (left) is a longitudinally sectional view of one of four longitudinally-long axially-inclined grooves (17a) circumferentially arranged around the feces sampling part (17) located on a front end side of the feces sampling stick (10). Figure 1 (right) shows three sectional views from a front end side toward a base end side of the feces sampling part (17). Figure 1 shows four longitudinally-long axially-inclined grooves (17a) circumferentially arranged on the feces sampling part (17) located on the front end side of the feces sampling stick (10). Each of the four longitudinally-long axially-inclined grooves (17a) is arranged in a spiral shape along the outer circumference of the feces sampling stick (10) from A (the front end side) toward B (the base end side) and thereby feces is easily pushed into the four longitudinally-long axially-inclined grooves (17a) along with rotation of the feces sampling stick (10). As shown in a longitudinally sectional view (left), a groove depth of the axially-inclined groove (17a) is gradually decreased from the front end side (17b) toward the base end side (17c).

Figure 1 also shows, immediately below the feces sampling part (17) of the feces sampling stick (10), a hemispherical part (13), a lower disk part (14), a circumferentially recessed part (15) that holds the feces, and an upper disk part (16) in sequence from the front end of the feces sampling stick (10).

Figure 2 is a perspective view of a longitudinal section of a leveling part (23) for feces in a cylindrical guiding part (22) of the feces sampling container. In inserting the feces sampling stick (not shown) into the cylindrical guiding part (22), the feces sampling stick is inserted from the top toward the bottom while being rotated. The feces, which has been sampled by the feces sampling part and adheres to the outer circumference of the feces sampling stick, can forcibly be pushed into the four longitudinally-long axially-inclined grooves (17a) of the feces sampling stick by a protruding member (24), eventually. The rotational direction of the feces sampling stick may be clockwise or counterclockwise. Note that in the embodiment illustrated in Figure 2, the rotational direction of the feces sampling stick is clockwise. The shape of the protruding member is not particularly limited, and the protruding member may have, for example, a structure that protrudes from an inner wall of the cylindrical guiding part toward the feces sampling stick while being inclined along the rotational direction of the feces sampling stick; or the protruding member may have a structure that protrudes stepwise from the inner wall of the cylindrical guiding part toward the feces sampling stick along the rotational direction of the feces sampling stick. In addition, the shape of the protruding member is not particularly limited, and the protruding member may have, for example, a structure that protrudes, along the axial direction of the feces sampling stick, from the inner wall of the cylindrical guiding part toward the feces sampling stick in such a manner as to protrude more as closer to the leveling part side, or the protruding member may have a structure that protrudes, along the axial direction of the feces sampling stick, from the inner wall of the cylindrical guiding part toward the feces sampling stick in such a manner as to protrude more as closer to the opposite side of the leveling part, or the protruding member may have a structure that protrudes from the inner wall of the cylindrical guiding part toward the feces sampling stick in such a manner as to protrude substantially parallel to the feces sampling stick. The shape of the protruding member in the rotational direction of the feces sampling stick and the shape of the protruding member in the axial direction of the feces sampling stick can be freely combined. In addition, in the embodiment shown in Figure 2, the shape of the protruding member has a structure that inclinedly protrudes from the inner wall of the cylindrical guiding part toward the feces sampling stick along the rotational direction of the feces sampling stick, and also a structure that protrudes along the axial direction of the feces sampling stick from the inner wall of the cylindrical guiding part toward the feces sampling stick and protrudes more as closer to the opposite side of the leveling part.

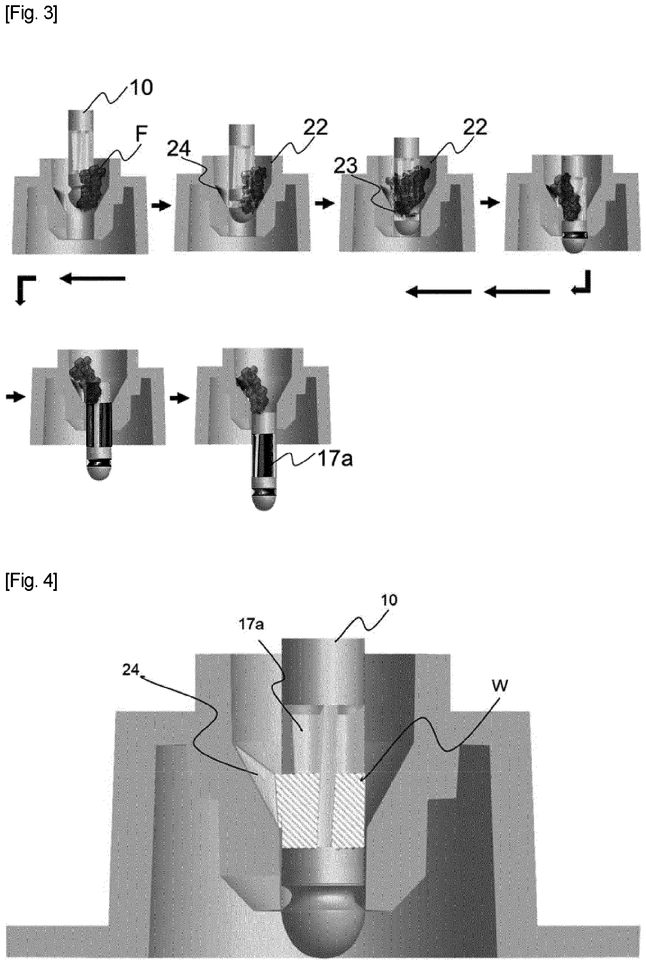

Figure 3 shows a function of feces filling by the protruding member (24). Feces (F) adhering near the front end of the feces sampling stick (10) is removed by the leveling part (23) along with insertion of the feces sampling stick (10). This excess feces thus removed remains at a boundary between the end on the leveling part side of the cylindrical guiding part (22) and the leveling part (23), and this remaining feces is accumulated on a side surface of the protruding member (24) along with the rotation of the feces sampling stick (10). At this time, further rotation of the feces sampling stick (10) eventually causes the protruding member (24) to fill the four longitudinally-long axially-inclined grooves (17a) circumferentially arranged on the feces sampling stick (10) with the feces.

Figure 4 shows a function capable of sampling feces even with a high moisture content, such as loose feces and watery feces (W). There is provided a structure that includes a small gap between the protruding member (24) and the feces sampling stick (10) so as to cause surface tension to act easily. Since a small gap is provided between the protruding member (24) and the feces sampling stick (10), the longitudinally-long axially-inclined grooves (17a) are naturally filled, due to the surface tension, with feces with a high moisture content, such as loose feces having an unstable shape due to its high moisture content and watery feces in a liquid state because of inability of maintaining the shape due to its high moisture content.

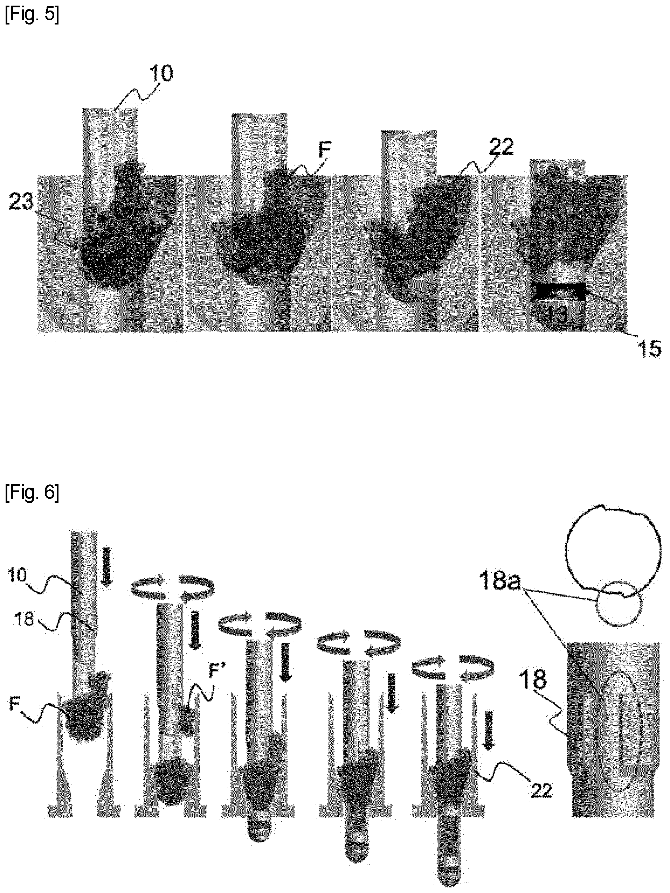

Figure 5 shows how the feces (F) at the front end of the feces sampling stick (10) moves when the feces (F) is sampled only by a part of the feces sampling stick (10) (the front end of the feces sampling part) . The following states are shown in sequence from the left in Figure 5: a state in which the feces (F) comes into contact with and is pulled by the leveling part (23); a state in which the feces (F) moves apart from the hemispherical part (13) as the feces sampling stick (10) is inserted because the hemispherical part (13) with a polished surface at the front end of the feces sampling stick (10) has a small coefficient of friction; a state in which the feces remains at a boundary between the end on the leveling part side of the cylindrical guiding part (22) and the leveling part (23); and a state in which the circumferentially recessed part (15) at the front end of the feces sampling stick (10) is filled with the feces.

Figure 6 (left) shows a function of each reduced-diameter step (18) having a cutout formed in the feces sampling stick (10) to scrape off the feces adhering to the inner wall of the cylindrical guiding part (22) by an axial edge (18a) of the cutout. The feces (F') adhering to the inner wall of the cylindrical guiding part (22) is scraped off by the axial edges (18a) of the cutouts and thereby the feces thus scraped-off drops to the boundary between the end on the leveling part side of the cylindrical guiding part (22) and the leveling part. Thereafter, the feces having dropped is then used as feces to be pushed into the longitudinally-long axially-inclined grooves (17a) together with the excess feces having been removed by the leveling part. Figure 6 (right) shows the axial edge (18a) of the cutout of the reduced-diameter step (18).

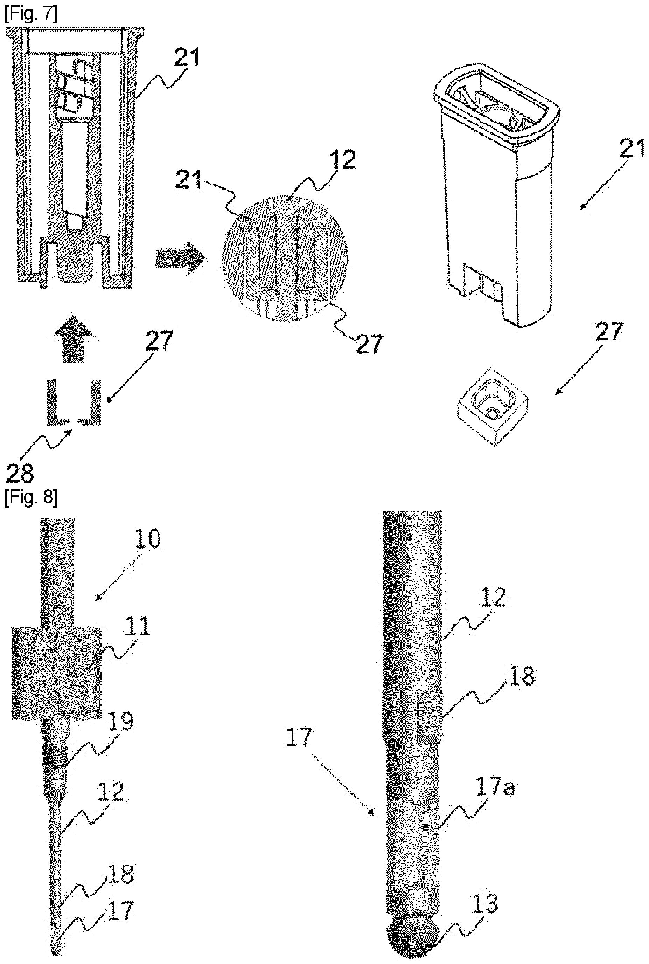

Figure 7 shows a detachable separator (27) made of PE or elastomer having a second leveling part (28). Figure 7 (upper left) is a longitudinally sectional view of a fitting body (21) made of PP, which is fitted into the container outer frame (not shown). Figure 7 (lower left) is a longitudinally sectional view of the detachable separator (27) made of PE or elastomer and including the second leveling part (28). Figure 7 (middle) is an enlarged view of a major part when the detachable separator (27) made of PE or elastomer is fitted to the bottom of the fitting body (21) made of PP. Figure 7 (upper right) is a perspective view of the fitting body (21) made of PP. Figure 7 (lower right) is a perspective view of the detachable separator (27) made of PE or elastomer. By using a PE material or an elastomer material softer than a PP material or other materials, while maintaining insertability of the feces sampling stick into the second leveling part (28), which is formed to have a hole diameter reduced to be not more than the diameter of the stick part (12) of the feces sampling stick, it is possible to enhance the function of removing the excess feces. The elastomer material for the separator is not particularly limited, and may be natural rubber, synthetic rubber, or resin elastomer, for example.

Figure 8 (left) shows the whole feces sampling stick, and Figure 8 (right) shows a round stick part of the feces sampling stick, respectively. As shown in Figure 8, the feces sampling stick (10) in the feces sampling container of one embodiment may include a gripping part (11) on one side and the stick part (12) on the other side, and may be provided with the feces sampling part (17) having the axially-inclined grooves (17a) near the front end of the stick part. A base end of the stick part (12) of the feces sampling stick (10) may be provided with a screw part (19) to be screwed into the container body (not shown). The stick part (12) is preferably a round stick part having a constant diameter with a circular cross section, and may be a regular polygon with a cross section close to a circle. Note that the stick part will be always referred to as a round stick part (12), hereinafter.

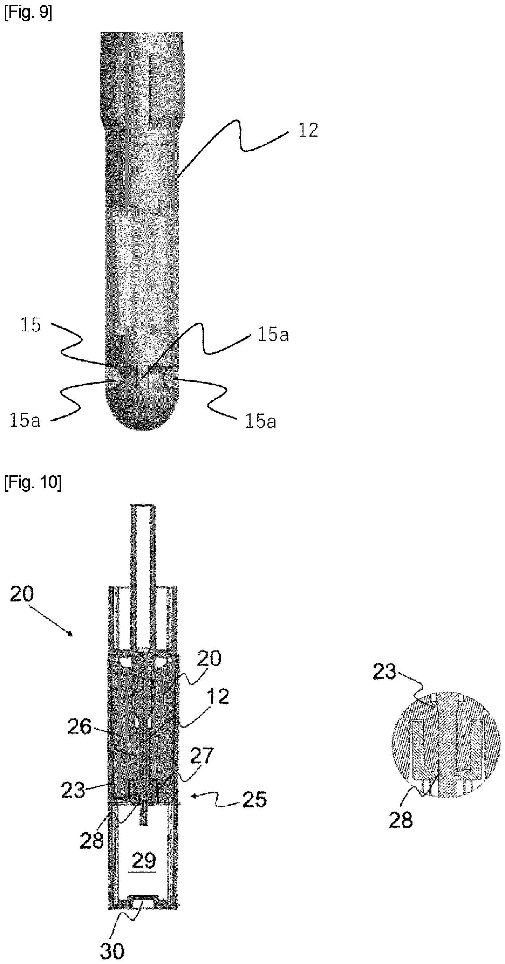

As shown in Figure 1 and Figure 8, the front end of the feces sampling stick (10) of one embodiment may include: the hemispherical part (13); the circumferentially recessed part (15) holding the feces; the upper disk part (16); and the feces sampling part (17) in sequence from the front end toward the base end side of the round stick part (12). In another embodiment, the lower disk part (14) may be provided between the hemispherical part (13) and the circumferentially recessed part (15). The lower limit of the axial thickness of the lower disk part (14) is not particularly limited, and may be, for example, 0.1 mm or more, 0.2 mm or more, 0.3 mm or more, 0.4 mm or more, 0.5 mm or more, 0.6 mm or more, 0.7 mm or more, 0.8 mm or more, 0.9 mm or more, 1.0 mm or more, 1.1 mm or more, 1.2 mm or more, 1.3 mm or more, 1.4 mm or more, 1.5 mm or more, 1.6 mm or more, 1.7 mm or more, 1.8 mm or more, 1.9 mm or more, or 2.0 mm or more. In addition, the upper limit of the axial thickness of the lower disk part (14) is not particularly limited, and may be, for example, 1.0 mm or less, 1.1 mm or less, 1.2 mm or less, 1.3 mm or less, 1.4 mm or less, 1.5 mm or less, 1.6 mm or less, 1.7 mm or less, 1.8 mm or less, 1.9 mm or less, 2.0 mm or less, 2.1 mm or less, 2.2 mm or less, 2.3 mm or less, 2.4 mm or less, 2.5 mm or less, 2.6 mm or less, 2.7 mm or less, 2.8 mm or less, 2.9 mm or less, or 3.0 mm or less. The above numerical values of the upper and the lower limits can be freely combined. The axial thickness of the lower disk part (14) is not particularly limited, and may be, for example, 0.1 mm or more and 3.0 mm or less, 0.5 mm or more and 1.5 mm or less, 1.0 mm or more and 2.0 mm or less, 1.5 mm or more and 2.5 mm or less, or 2.0 mm or more and 3.0 mm or less. The hemispherical part (13) may have a polished surface. In addition, the circumferentially recessed part (15) may be provided with a rib or ribs. The shape of the rib is not particularly limited, and for example, as shown in Figure 9, may have a shape that only partially interrupts the recessed part of the circumference of the circumferentially recessed part (15), or may have a shape that extendingly provides the ribs in the axial direction. The number of the ribs is not particularly limited, and may be, for example, 1, 2, 3, 4, 5, 6, 7 or 8. Figure 9 shows the stick part (12) of the feces sampling stick having four ribs (15a) provided every 90° in the circumferentially recessed part (15) .

In one embodiment, the feces sampling part (17) of the feces sampling stick (10) may be provided with the axially-inclined grooves (17a) into which the feces is easily pushed along with the rotation of the feces sampling stick (10), and the axially-inclined grooves (17a) may have a spiral structure extending from the front end toward the base end along the outer circumference of the feces sampling stick (10). The number of the axially-inclined grooves (17a) is not particularly limited, and may be, for example, 1, 2, 3, 4, 5, 6, 7 or 8. Note that in the embodiment shown in Figure 1, the feces sampling part (17) having four axially-inclined grooves (17a) is illustrated. In addition, the lower limit of a distance from one end to the other end in the axial direction of each axially-inclined groove (17a) is not particularly limited, and may be, for example, 1.0 mm or more, 1.1 mm or more, 1.2 mm or more, 1.3 mm or more, 1.4 mm or more, 1.5 mm or more, 1.6 mm or more, 1.7 mm or more, 1.8 mm or more, 1.9 mm or more, 2.0 mm or more, 2.1 mm or more, 2.2 mm or more, 2.3 mm or more, 2.4 mm or more, 2.5 mm or more, 2.6 mm or more, 2.7 mm or more, 2.8 mm or more, 2.9 mm or more, 3.0 mm or more, 3.1 mm or more, 3.2 mm or more, 3.3 mm or more, 3.4 mm or more, 3.5 mm or more, 3.6 mm or more, 3.7 mm or more, 3.8 mm or more, 3.9 mm or more, or 4.0 mm or more. Further, the upper limit of the distance from one end to the other end in the axial direction of each axially-inclined groove (17a) is not particularly limited, and may be, for example, 2.0 mm or less, 2.1 mm or less, 2.2 mm or less, 2.3 mm or less, 2.4 mm or less, 2.5 mm or less, 2.6 mm or less, 2.7 mm or less, 2.8 mm or less, 2.9 mm or less, 3.0 mm or less, 3.1 mm or less, 3.2 mm or less, 3.3 mm or less, 3.4 mm or less, 3.5 mm or less, 3.6 mm or less, 3.7 mm or less, 3.8 mm or less, 3.9 mm or less, 4.0 mm or less, 4.1 mm or less, 4.2 mm or less, 4.3 mm or less, 4.4 mm or less, 4.5 mm or less, 4.6 mm or less, 4.7 mm or less, 4.8 mm or less, 4.9 mm or less, or 5.0 mm or less. The above numerical values of the upper and the lower limits can be freely combined. The distance from one end to the other end in the axial direction of each axially-inclined groove (17a) is not particularly limited, and may be, for example, 1.0 mm or more and 5.0 mm or less, 1.0 mm or more and 2.5 mm or less, 1.5 mm or more and 3.0 mm or less, 2.0 mm or more and 3.5 mm or less, or 2.5 mm or more and 4.0 mm or less.

In one embodiment, the groove depth of each axially-inclined groove (17a) may be gradually decreased from the front end side (17b) toward the base end side (17c). The lower limit of the depth of the axially-inclined groove (17a) is not particularly limited, and may be, for example, 0.10 mm or more, 0.11 mm or more, 0.12 mm or more, 0.13 mm or more, 0.14 mm or more, 0.15 mm or more, 0.16 mm or more, 0.17 mm or more, 0.18 mm or more, 0.19 mm or more, 0.20 mm or more, 0.21 mm or more, 0.22 mm or more, 0.23 mm or more, 0.24 mm or more, 0.25 mm or more, 0.26 mm or more, 0.27 mm or more, 0.28 mm or more, 0.29 mm or more, 0.30 mm or more, 0.31 mm or more, 0.32 mm or more, 0.33 mm or more, 0.34 mm or more, 0.35 mm or more, 0.36 mm or more, 0.37 mm or more, 0.38 mm or more, 0.39 mm or more, or 0.40 mm or more. The upper limit of the depth of the axially-inclined groove (17a) is not particularly limited, and may be, for example, 0.30 mm or less, 0.31 mm or less, 0.32 mm or less, 0.33 mm or less, 0.34 mm or less, 0.35 mm or less, 0.36 mm or less, 0.37 mm or less, 0.38 mm or less, 0.39 mm or less, 0.40 mm or less, 0.41 mm or less, 0.42 mm or less, 0.43 mm or less, 0.44 mm or less, 0.45 mm or less, 0.46 mm or less, 0.47 mm or less, 0.48 mm or less, 0.49 mm or less, or 0.50 mm or less. The above numerical values of the upper and the lower limits can be freely combined. The depth of the axially-inclined groove (17a) is not particularly limited, and may be, for example, 0.10 mm or more and 0.50 mm or less, 0.10 mm or more and 0.20 mm or less, 0.20 mm or more and 0.30 mm or less, 0.30 mm or more and 0.40 mm or less, or 0.40 mm or more and 0.50 mm or less.

In this case, the lower limit of a difference between a shallow portion and a deep portion of the groove is not particularly limited, and may be, for example, 0.01 mm or more, 0.02 mm or more, 0.03 mm or more, 0.04 mm or more, 0.05 mm or more, 0.06 mm or more, 0.07 mm or more, 0.08 mm or more, 0.09 mm or more, or 0.10 mm or more. The upper limit of the difference between the shallow portion and the deep portion of the groove is not particularly limited, and may be, for example, 0.11 mm or less, 0.12 mm or less, 0.13 mm or less, 0.14 mm or less, 0.15 mm or less, 0.16 mm or less, 0.17 mm or less, 0.18 mm or less, 0.19 mm or less, or 0.20 mm or less. The above numerical values of the upper and the lower limits can be freely combined. The difference between the shallow portion and the deep portion of the groove is not particularly limited, and may be, for example, 0.01 mm or more and 0.20 mm or less, 0.01 mm or more and 0.10 mm or less, or 0.01 mm or more and 0.05 mm or less.

In one embodiment, the spiral structure of the axially-inclined grooves (17a) extending from the front end side (A) toward the base end side (B) along the outer circumference of the feces sampling stick (10) may be either clockwise or counterclockwise from the front end side (A) toward the base end side (B) of the round stick part (12), and this spiral structure is preferably in the same direction as the above-described rotational direction of the feces sampling stick. The lower limit of an angle of the spiral is not particularly limited, and may be, for example, 1° or more, 2° or more, 3° or more, 4° or more, 5° or more, 6° or more, or 7° or more. The upper limit of the angle of the spiral is not particularly limited, and may be, for example, 3° or less, 4° or less, 5° or less, 6° or less, 7° or less, 8° or less, 9° or less, or 10° or less. The above numerical values of the upper and the lower limits can be freely combined. The angle of the spiral is not particularly limited, and the spiral may have a spiral structure having an angle of the spiral of 1° or more and 10° or less, 1° or more and 2° or less, 2° or more and 3° or less, 3° or more and 4° or less, 4° or more and 5° or less, 5° or more and 6° or less, or 6° or more and 7° or less for example.

In one embodiment, in order to provide a function of scraping off the feces at the center part of the feces sampling container, the reduced-diameter steps (18) with the cutouts may be provided to the base end side of the round stick part (12) of the feces sampling stick (10). The number of the reduced-diameter steps (18) with the cutouts is not particularly limited, and may be, for example, 1, 2, 3 or 4.

The gripping part (11) provided on one side of the feces sampling stick (10) of one embodiment may have a shape functioning as a cap member of the feces sampling container to prevent leakage or splashing of the fecal solvent before the feces sampling operation and leakage or splashing of a fecal solution in which the feces is suspended in the fecal solvent after the feces sampling operation; and for example, the lower end of the gripping part (11) may be formed in a flange shape or a skirt shape, or the gripping part may be subjected to an appropriate surface processing for facilitating gripping at the time of the feces sampling. In addition, in order to provide a liquid-tight function to the feces sampling stick (10) having the gripping part (11) as a cap member, the feces sampling stick (10) may be screwed and fitted into the fitting body (21) in a liquid-tight manner through the screw part (19) provided to the base end of the round stick part (12) of the feces sampling stick (10). In addition, the rotation pitch when the feces sampling stick (10) is screwed into the fitting body (21) is not particularly limited, and this pitch may be set such that a ratio between an insertion distance of the feces sampling stick (10) and a distance from the base end side (17c) to the front end side (17b) of the axially-inclined groove (17a) is 1:1 when the feces sampling stick (10) is screwed in by 1 turn or more, 1.5 turns or more, 2 turns or more, 2.5 turns or more, 3 turns or more, 3.5 turns or more, 4 turns or more, 4.5 turns or more, or 5 turns or more for example. In this case, the distance from the base end side (17c) to the front end side (17b) of the axially-inclined groove (17a) may be the above distance from one end to the other end in the axial direction of the axially-inclined groove (17a).

The lower limit of the diameter of the round stick part (12) in the base end side (17c) of the axially-inclined groove (17a), the upper disk part (16), or the lower disk part (14) is not particularly limited, and may be, for example, 1.0 mm or more, 1.1 mm or more, 1.2 mm or more, 1.3 mm or more, 1.4 mm or more, 1.5 mm or more, 1.6 mm or more, 1.7 mm or more, 1.8 mm or more, 1.9 mm or more, or 2.0 mm or more. The upper limit of the diameter of the round stick part (12) in the base end side (17c) of the axially-inclined groove (17a), the upper disk part (16), or the lower disk part (14) is not particularly limited, and may be, for example, 2.0 mm or less, 2.1 mm or less, 2.2 mm or less, 2.3 mm or less, 2.4 mm or less, 2.5 mm or less, 2.6 mm or less, 2.7 mm or less, 2.8 mm or less, 2.9 mm or less, or 3.0 mm or less. The above numerical values of the upper and the lower limits can be freely combined. The diameter of the round stick part (12) in the base end side (17c) of the axially-inclined groove (17a), the upper disk part (16), or the lower disk part (14) is not particularly limited, and may be, for example, 1.0 mm or more and 3.0 mm or less, 1.0 mm or more and 2.0 mm or less, 1.5 mm or more and 2.5 mm or less, or 2.0 mm or more and 3.0 mm or less.

The length of the stick part of the feces sampling stick (10) of one embodiment may be a length long enough for the feces sampling part (17) near the front end of the round stick part (12) to pass through the second leveling part (28) provided to the fitting body (21) or the separator (27). In addition, when the fecal solvent is stored in the fecal solvent storing part (29) described later, the length of the stick part of the feces sampling stick (10) may be a length long enough for the feces sampling part (17) near the front end of the round stick part (12) of the feces sampling stick (10) to pass through the leveling part (23) and the second leveling part (28) and be soaked into the fecal solvent. The material of the feces sampling stick (10) is not particularly limited, and may be, for example, low-density PE, ABS resin, or the like.

Figure 10 (left) is a longitudinally sectional view of the feces sampling container of a "fecal solvent type" that includes: the feces sampling stick; the container body including the container outer frame and the fitting body to be fitted inside the container outer frame; and the fecal solvent storing part. In addition, Figure 10 (right) is an enlarged view of a major part around the leveling part and the second leveling part.

The container body (20) of one embodiment may be separatable into a plurality of members. When the container body is separatable into a plurality of members, the cylindrical guiding part (22) and the leveling part (23) may be collectively disposed as one member among the plurality of members, or may be separately arranged as the plurality of members. The number or type of the plurality of members is not particularly limited, and these members may be, for example, the container outer frame (25) and the fitting body (21) fitted inside this container outer frame, and in this case, the cylindrical guiding part (22) and the leveling part (23) may be disposed to the fitting body (21). The container outer frame (25) is not particularly limited as long as this is a bottomed cylindrical container having an opening used for inserting the fitting body (21) on one side and a bottom on the other side, and may be a bottomed cylindrical container having a cross section in a square, rectangle, oval, circle, or other shapes. Further, in order to securely guide a front end of a fecal solution suction nozzle to a piercing part so as to reliably perform the sampling, the bottom of the container body (20) may be formed to be a concave bottom (30) with the piercing part, especially a tapered concave bottom gradually tapered toward the bottom. The concave bottom (30) with the piercing part may have a strength or a structure capable of being pierced by the front end of the fecal solution suction nozzle. The fecal solvent may be stored in advance in the fecal solvent storing part (29) formed between a lower part of the container outer frame (25) and below the fitting body (21).

The material of the container body (20) of one embodiment is not particularly limited, and may be, for example, a flexible resin such as PP, PE, polyester, or polyvinyl chloride. The fecal solvent is not particularly limited as long as this is a solution in which the feces sampled by the feces sampling part of the feces sampling stick can be suspended, and may be, for example, aqueous medium, such as deionized water, distilled water, and a buffer solution. The buffer solution is not particularly limited, and may be, for example, a phosphate buffer solution, a carbonate buffer solution, an ammonia buffer solution, an acetate buffer solution, a lactate buffer solution, a citrate buffer solution, a tartrate buffer solution, a borate buffer solution, a glycine buffer solution, or a Good's buffer solution.

The fitting body (21) fitted inside the container outer frame (25) of one embodiment can isolate the inside of the container body (20) in a liquid-tight manner into an upper part and the fecal solvent storing part (29) below, and may be provided with the cylindrical guiding part (23) that allows the round stick part (12) of the feces sampling stick (10) to be inserted therein. The inside of the cylindrical guiding part (22) may be partially or entirely provided with a reduced-diameter portion (reverse-tapered structure) that is gradually reduced in diameter toward the insertion direction of the round stick part (12). The leveling part (23) for removing the excess feces may be provided at a lower end of the reduced-diameter portion of the cylindrical guiding part (22). Further below the leveling part (23) of the cylindrical guiding part (22), a thin cylindrical part (26), and the second leveling part (28) provided to the separator (27) may be provided in sequence.

In one embodiment, the protruding member (24) may be provided on the inner wall at a lower part of the reduced-diameter portion of the cylindrical guiding part (22) (the end on the leveling part side of the cylindrical guiding part). In one embodiment, a predetermined gap may be provided between the protruding member (24) and the round stick part (10), as described above.

In one embodiment, the feces sampled by the feces sampling stick (10) is passed through the leveling part (23) and the second leveling part (28) to remove excess feces therefrom, and thereafter is suspended in the fecal solvent stored in the fecal solvent storing part (29), to thereby provide the fecal solution. The second leveling part (28) may be provided together with the leveling part (23) to the fitting body (21), and may be formed as the PE or elastomer separator (27) that can be fitted to the bottom of the fitting body (21).

The material used for forming the fitting body (21) of one embodiment is not particularly limited, and may be a soft flexible resin such as PE, PP, polyester, soft polyvinyl chloride, and olefinic elastomer, considering the liquid-tight sealability, insertion and fitness into the container outer frame (25), insertability of the feces sampling stick (10) through the leveling part (23) and the second leveling part (28).

A method for collecting feces of one embodiment using the above-described feces sampling container of one embodiment will be exemplified. The feces sampling stick (10) is removed from the feces sampling container, and the feces sampling part (17) of the feces sampling stick (10) is sticked into feces or rubbed against the surface of the feces to collect the feces. Subsequently, the round stick part (12) of the feces sampling stick (10) is inserted into the container body (20) in an upright state through the cylindrical guiding part (22) of the fitting body (21), and the gripping part (11) of the feces sampling stick (10) is then screwed in such that the screw part (19) provided to the base end of the stick part of the feces sampling stick (10) is screwed into a screw hole provided to the upper part of the fitting body to seal the container body (20), and at the same time, the feces sampling part (17) of the feces sampling stick (10) is inserted through the leveling part (23) and the second leveling part (28) to be soaked in the fecal solvent stored in advance in the fecal solvent storing part (29) .

As described above, the feces sampling container of a "fecal solvent type" (see Patent Document 1), which is one embodiment of the present invention, has been described above; and as another embodiment, for example, the present invention may be applied to a feces sampling container of a "fecal desiccant type" as described in Patent Document 3. That is, in one embodiment, the fecal solvent storing part of the container body described above may be used as a desiccant storing part. In this case, the method may be configured such that a feces sample after the feces sampling adhering to the feces sampling part of the feces sampling stick is brought into contact with a desiccant in the desiccant storing part so as to dry this feces sample; and after the feces sample is stored in a dried state in the desiccant, an aqueous medium is added to the desiccant where the feces sample in a dried state is stored so as to dissolve components in the feces sample into this aqueous medium, and then the components dissolved in the aqueous medium are measured. The desiccant is not particularly limited as long as this can dry the feces sample, and may be, for example, silica gel or aluminum oxide.

### Industrial Applicability

The present disclosure provides a feces sampling container and a method for collecting feces that are useful for a colon cancer screening or the like.

### Explanation of Letters or Numerals

- 10: feces sampling stick
- 11: gripping part of feces sampling stick
- 12: stick part of feces sampling stick
- 13: hemispherical part
- 14: lower disk part
- 15: circumferentially recessed part
- 15a: rib provided to circumferentially recessed part
- 16: upper disk part
- 17: feces sampling part of feces sampling stick
- 17a: axially-inclined groove
- 17b: front end side of axially-inclined groove
- 17c: base end side of axially-inclined groove
- 18: reduced-diameter step having cutout
- 18a: axial edge of cutout of reduced-diameter step
- 19: screw part at base end of stick part of feces sampling stick
- 20: container body
- 21: fitting body
- 22: cylindrical guiding part
- 23: leveling part
- 24: protruding member
- 25: container outer frame
- 26: thin cylindrical part
- 27: separator
- 28: second leveling part
- 29: fecal solvent storing part
- 30: concave bottom having piercing part
- F: excess feces adhering to feces sampling part
- F': feces adhering to inner wall of cylindrical guiding part
- W: loose feces or watery feces

## Claims

1. A feces sampling container comprising:
a feces sampling stick that includes a stick part and a feces sampling part having an axially-inclined groove near an end of the stick part; and
a container body that houses the feces sampling stick and includes a leveling part to scrape off feces at a position through which the feces sampling part passes when housing the feces sampling stick in the container body.

2. The feces sampling container according to claim 1,
wherein
the stick part of the feces sampling stick has a screw part, and
the container body includes a screw hole into which the screw part is screwed.

3. The feces sampling container according to claim 2, wherein the feces sampling stick includes the screw part on a base portion of the other end of the stick part.

4. The feces sampling container according to any one of claims 1 to 3, wherein
when the feces sampling stick is screwed into the container body by 1.5 turns or more, a ratio of a distance of insertion of the feces sampling stick to a distance from one end to the other end in an axial direction of the axially-inclined groove is 1:1.

5. The feces sampling container according to any one of claims 1 to 4, further comprising a plurality of axially-inclined grooves.

6. The feces sampling container according to any one of claims 1 to 5, wherein a groove depth of the axially-inclined groove is gradually decreased from a front end side toward a base end side of the stick part.

7. The feces sampling container according to any one of claims 1 to 6, wherein the axially-inclined groove has a spiral structure at an angle of 3° or more and 4° or less along an outer circumference of the stick part.

8. The feces sampling container according to any one of claims 1 to 7, wherein the container body includes a cylindrical guiding part that guides the feces sampling part to the leveling part, and the cylindrical guiding part includes a protruding member on an inner wall of an end on a leveling part side of the cylindrical guiding part.

9. The feces sampling container according to any one of claims 1 to 8, wherein the stick part is a round stick part having a constant diameter, and the round stick part includes: a hemispherical part; a circumferentially recessed part; and an upper disk part sequentially from a front end side toward a base end side of the round stick part.

10. The feces sampling container according to claim 9, wherein the hemispherical part has a polished surface.

11. The feces sampling container according to claim 9 or 10, wherein the circumferentially recessed part includes one or more ribs.

12. The feces sampling container according to any one of claims 9 to 11, wherein the stick part includes a lower disk part between the hemispherical part and the circumferentially recessed part.

13. The feces sampling container according to claim 12, wherein an axial thickness of the lower disk part is 0.5 mm or more and 1.5 mm or less.

14. The feces sampling container according to any one of claims 9 to 13, wherein the feces sampling container has a gap between the protruding member and the round stick part.

15. The feces sampling container according to any one of claims 9 to 14, wherein the round stick part includes a reduced-diameter step having a cutout on a base end side of the round stick part.

16. The feces sampling container according to any one of claims 9 to 15, further comprising a second leveling part formed so that a hole diameter is reduced to be not more than a diameter of the round stick part.

17. The feces sampling container according to claim 16, wherein:
the container body includes a container outer frame and a fitting body fitted inside the container outer frame;
the fitting body includes a separator made of a polyethylene (PE) or elastomer material, the separator fittable to a bottom of the fitting body; and
the separator includes the second leveling part.

18. A method for collecting feces comprising collecting feces using the feces sampling container according to any one of claims 1 to 17.
